# EUROPEAN PATENT APPLICATION

(11) **EP 1 990 062 A1**
(43) Date of publication of application: **12.11.2008**
(21) Application number: 07714596.9
(22) Date of filing: 20.02.2007
(51) Int. Cl.: A61K 47/04, A61K 9/16, A61K 9/20, A61K 47/10, A61K 47/26, A61K 47/36, A61K 47/44, A61K 47/46

(54) **GRANULES, TABLETS AND METHOD OF PRODUCING THE SAME**

(30) Priority: 20.02.2006 JP 2006042906
(71) Applicant: ASAHI BREWERIES, Ltd., Sumida-ku Tokyo 130-8602 (JP)
(72) Inventor: HIRAI, Nobuaki, Moriya-shi, Ibaraki 3020106 (JP); ISHIKAWA, Kazuyuki, Tokyo 1300001 (JP)
(74) Representative: Feldkamp, Rainer
(86) International application number: PCT/JP2007/053093
(87) International publication number: WO 2007/097333

(57) **Abstract**

When a light and porous inorganic substance is employed in a large amount as a excipient, it is impossible in the subsequent tabletting step using a tabletting machine to supply a desired amount of the resultant powder into a mortar having a definite diameter. As a result, extremely light tablets are produced. A method of producing the tablet is **characterized by** including dissolving or dispersing an oily substance, a liquid substance derived from a natural product or a low-melting substance in an organic solvent, allowing a porous calcium silicate powder to adsorb and hold the solution, adding a starch and/or a saccharide thereto, granulating the obtained mixture to give granules having an appropriate loose bulk density, and then compression molding those granules to give tablets.

## Description

### Technical Field

The present invention relates to granules, tablets, each including an oily substance, a liquid extract derived from a natural product, or a low-melting substance, and to a method of producing the same.

### Background Art

As formulations for taking a functional material, there are several formulation of powders, granules, capsules, tablets, and the like. In general, the tablet formulation is used most frequently because of its easy handling and ingestion.
An oily substance, a liquid substance derived from natural product, and also a low-melting substance frequently found in functional materials are dissolved or dispersed in edible oils, and then formed into preparations by packing the resulting substances of them in soft capsules, and this method is a main stream of preparations.

As the material of soft capsules, gelatins derived from animals are mainly used. However, due to the problem of BSA (BSE; Bovine Spongiform Encephalopathy), it has recently become difficult to use the gelatin derived from bovines. Then, while gelatins of pigs, fishes, and the like instead of bovines are used, it is difficult for those gelatins to replace the bovine gelatin.

If the oily substance and the liquid substance derived from natural product can be formed into powders, and if physical properties of the low-melting substance can be improved, it becomes possible to form those substances into tablets without using soft capsules.
Then, there is an art of forming tablets by adsorbing an oily component to calcium silicate (for example, see Patent Document 1).
In general, tablets are produced by adding water and/or an organic solvent to a mixed powder compounded of an appropriate component such as a functional material, a excipient, a binder, or a disintegrant (all of which are polymer additives derived from cellulose, a synthetic product, or a natural product) to granulate the mixed powders, pouring the obtained granulated granules in a certain quantity on a turntable of a tabletting machine from a hopper (a container containing the granulated granules), and compressing the granulated granules in a hole (mortar) formed on the turntable with two pairs of top and bottom steel bars (a top rod and a bottom rod).

In the above production, the hole (mortar) is a cylinder with a definite diameter, so the weight of the packed granules therein and the balance between the diameter and thickness of the obtained tablet are important. The packed amount is controlled by a loose bulk density of granules. When the value thereof is excessively small, a mortar with an extremely large diameter is needed to obtain a tablet having a definite weight. As a result, a flat tablet with a large diameter is obtained, so the ingestion of the tablet is difficult and also, the tablet is easily cracked. In addition, on the contrary, when the value is excessively large, the tablet with a small diameter is obtained, resulting in difficulty in handling.

A tablet with a diameter of 6 to 10 mm is generally recommended, whereby it is important for granules to have a loose bulk density of 0.3 to 0.8 g/ml. Besides, fluidity is important property for granules to flow in a mortar smoothly.
In addition, when granules contain an oily substance and a liquid substance derived from natural product in a large amount, because those materials are liquid at normal temperature, the compressibility of the granules is impaired. Accordingly, it is important to produce granules excellent in compressibility by preventing the oily substance and the liquid substance derived from natural product from liberating from the granules.

In addition, as the tabletting trouble in granules containing a low-melting substance in a large amount, there is the sticking that the granules are melted during tabletting due to mechanical friction between the rod and the mortar, resulting in adhesion of the melted substance to the rod. The property for avoiding the tabletting trouble such as the above phenomenon is also important property demanded for granules.
As described above, various additives are used in order to impart characters for formation into a tablet to the granulated granules.

As the additives, there are excipients, binders, disintegrants, lubricants, and the like depending on the use of the additives.
As the excipients, saccharides such as lactose and maltitol, sugar alcohols, starches such as a corn starch and a potato starch, and crystalline cellulose are used in many cases. In addition, it is also known that, as other excipients, an inorganic substance such as a light anhydrous silicic acid, calcium silicate, or calcium phosphate is used. Of those, in drugs, it is known that, when a porous inorganic substance is used, the porous inorganic substance is used as a carrier of a pharmaceutical, and the pharmaceutical in a state of being dissolved in an organic solvent or dissolved by heat in the absence of an organic solvent is mixed with the porous inorganic substance so as to adsorb to the porous inorganic substance, which is used to hold the pharmaceutical. However, the porous inorganic substance has a small loose bulk density, and there is a problem that the tablet becomes excessively large when produced using the porous inorganic substance. For example, in a case of calcium silicate having a loose bulk density of 0.1 g/ml, only a tablet having a weight of 100 mg or less is produced even when the calcium silicate is packed in a mortar with a diameter of 10 mm. For example, in order to produce a tablet having a weight of 400 mg, a mortar with a diameter of 15 mm or more is needed, whereby the tablet becomes excessively large, flat, and easily cracked.
Patent Document 1: JP 08-157362 A

### Disclosure of the Invention

### Problem to be solved by the Invention

According to the conventional art described above, when powders in which an oily component is adsorbed to calcium silicate is prepared, the substance mixed with the powders generally has a loose bulk density of as small as about 0.2 g/ml. With the tabletting powders having such loose bulk density, only extremely little amount of adsorbed powders are loaded into a mortar hole in a step of supplying a definite amount of powders into the mortar hole during the tabletting step. When the powders are tableted, an extremely thin tablet is obtained, and there is a problem that the tablet can not be used practically.

In a case of producing a tablet including an oily substance and a liquid extract derived from a natural product, when granules including those substances are compressed with a tabletting machine under high pressure, an oil and fat substance and the like liberate from granules, with the result that compressibility of the granules are impaired and a tablet having hardness for practical use can not be obtained. For example, when granules having a large amount of extract content derived from *Serenoa repens* (saw palmetto) being liquid at normal temperature is compressed, the *Serenoa repens* extract is desorped from the granules, whereby a tablet having sufficient hardness can not be produced.

In addition, in a case when tablets having a low-melting substance are produced, the low-melting substance is stuck to surfaces of mortar and rods of a tabletting machine. As a result, produced tablets fluctuate in weight, and irregularities are caused on surfaces of the tablets, thereby being difficult to attain stable production of tablets. This is because, if the tabletting is performed continuously, the low-melting substance is caused to melt due to increases in the surface temperatures of the mortar and rods of the tabletting machine.
For example, though coenzyme Q10 is a solid substance at normal temperature, because the melting point of coenzyme Q10 is as low as 48°C, the sticking phenomenon occurs vigorously in producing tablets continuously with the tabletting machine. Accordingly, production of tablets including the substance in a large amount becomes difficult.

It is predicted that the compressibility and the sticking phenomenon can be alleviated to some extent by using a porous inorganic substance as a excipient and allowing its pores to adsorb and hold a functional substance. However, there are differences in effects depending on the kind of porous inorganic substances to be used, the substance to be adsorbed, and a method for adsorption, and hence the combination with which the compressibility and the sticking phenomenon can be highly alleviated at a sufficient level is required.
In addition, there are many porous inorganic substances which can not be granulated by themselves because of their lightness (small loose bulk density) such as calcium silicate powders.

When the light porous inorganic substance is used in a large amount, even if supplied in a tabletting machine in the subsequent step of tabletting, an intended amount of powders can not be supplied in a mortar with a definite diameter. As a result, an extremely light tablet is obtained.
Therefore, an object of the present invention is to obtain a tablet having such an appropriate loose bulk density that compressibility of granules is highly alleviated, the sticking phenomenon upon tabletting is highly avoided, and a tablet has an appropriate size and weight in producing a tablet including an oily substance and a liquid extract derived from a natural product, which are formed into preparation with a soft capsule, or including a low-melting substance which is formed into preparation with soft capsules in many cases and induces a tabletting obstacle.

### Means for solving the Problems

Therefore, the present invention is granulated by using porous calcium silicate powders as a excipient, allowing the porous calcium silicate to adsorb and hold an oily substance, a liquid substance derived from a natural product, or a low-melting substance in a state of being dissolved or dispersed in an organic solvent, and adding a starch or a saccharide as a heavy substance in combination.
That is, the present invention relates to a method including allowing porous calcium silicate powders to adsorb and hold a solution in which an oily substance, a liquid substance derived from a natural product, or a low-melting substance is dissolved or dispersed in an organic solvent, then adding a starch and/or a saccharide thereto, and granulating the resultant solution to produce granules having an appropriate loose bulk density, and also relates to a tablet obtained by compression molding the granules.

### Effects of the Invention

The present invention as described above enables production of a tablet having an appropriate hardness at low pressures, including an oily substance and a liquid substance derived from a natural product that adversely effect on the compressibility. In addition, the present invention enables production of a tablet including a low-melting substance which is likely to cause the sticking phenomenon upon tabletting in a state where the sticking phenomenon is avoided.
In addition, even when porous calcium silicate powders are used as a excipient, granules having an appropriate loose bulk density can be obtained, and also, a tablet with a more appropriate diameter and weight can be obtained by compression molding the granules.

The present invention has an effect of producing intended tablets which have no variation in weight, each have a flat surface, and include an oily substance, a liquid substance derived from natural product, and a low-melting substance.
In addition, in spite of use of porous calcium silicate, an effect of producing a tablet including 50 mg or more of the functional substances, the tablet having a diameter of 8 mm and a weight of 300 mg, can be obtained.

### Best Mode for carrying out the Invention

In the present invention, the oily substance denotes a substance which is liquid at normal temperature (20°C). For example, oily substance includes vitamin E, vitamin A, docosahexaenoic acid, eicosapentaenoic acid, and the like.
The liquid substance derived from a natural product includes a substance extracted from a natural product, and collectively designates a liquid substance of which dried substance can not be formed because a large amount of oily extracts mainly formed of a lipid-soluble component or because an organic acid such as acetic acid, which can not be crystallized, is included therein. For example, liquid substance derived from a natural product includes products obtained by subjecting a *Serenoa repens* extract, products of lactic fermentation from a vegetable juice, black vinegar, and the like.

The low-melting substance denotes a substance which has a melting point of 120°C or lower and is solid at normal temperature (20°C). From the viewpoint of occurrence of the sticking phenomenon, the melting point is preferably 100°C or lower. For example, low-melting substance includes coenzyme Q10, α-lipoic acid, and riboses.
The calcium silicate powders which hold such an oily substance, a liquid substance derived from a natural product, or a low-melting substance (each of which may be referred as functional substance in this specification), are porous powders. In general, it is preferred that the calcium silicate powders have an average diameter of 18 to 32 µm, a loose bulk density of 0.007 to 0.015 g/ml, and oil absorption of 300 to 550 ml/100 g. Examples of the calcium silicate powders having the above properties include gyrolite type calcium silicate powders represented by the following chemical formula,

2CaO·3SiO₂·mSiO₂·nH₂O

(In the formula, 1 < m < 2, 2 < n < 3)
and having a petaloid crystal structure when observed with an electron microscope. Specifically, there is FLORITE (trade name) manufactured by TOKLTYAMA Corp.

The functional substance is adsorbed to and held by the porous calcium silicate powers by mixing the calcium silicate powders and the functional substance in a solution state where the functional substance is dissolved or dispersed in an organic solvent.
According to the adsorption and hold of the functional substance in a state of being dissolved or dispersed in an organic solvent, the functional substance is densely held by fine pores of calcium silicate powders.

Therefore, the functional substance does not effuse on the granule surfaces, so the granules are excellent in compressibility and the sticking phenomenon is largely avoided upon tabletting.
However, even when the porous calcium silicate is used as an excipient, favorable compression moldability can not be simply obtained by only direct mixing of the porous calcium silicate with the oily substance or the liquid substance derived from a natural product. In addition, even by direct mixture of the low-melting substance and the calcium silicate as well, a suppressive effect of the sticking phenomenon is hardly exhibited.

As an organic solvent to be used in dissolving the functional substance, ethanol is preferred solvent. In addition, a little amount of water may be mixed together with ethanol.
The organic solvent in which the functional substance is dissolved or dispersed may be mixed with the porous calcium silicate powders in any containers. In general, it is preferred to mix the organic solvent and powders under agitation using a mixer, a mixer granulator used in the granulating step described later, or the like□

As the amount of the organic solvent in which the functional substance is dissolved or dispersed, it is sufficient to the amout of the organic solvent in which the functional substance has been dissolved or dispersed. When the additional amount of organic solvent is excessively small, the functional substance is not adsorbed uniformly. On the other hand, when the additional amount of organic solvent is excessively large, the functional substance gets moistness. Therefore, the porous calcium silicate powders are mixed in an amount of 50 to 300 parts by weight and preferably 100 to 200 parts by weight with respect to 100 parts by weight of the functional substance.
In view of the foregoing, a functional substance-holding calcium silicate powders, which the functional substance is adsorbed to and held by, can be prepared.

Note that the organic solvent may be removed from the functional substance-holding calcium silicate powders in this step, but it is efficient to remove the organic solvent in the drying step after granulation.
The thus obtained functional substance-holding calcium silicate powders are formed into granules by adding a starch, a saccharide, or a mixture of them and then granulating the resultant.
It is difficult to granulate the functional substance-holding calcium silicate powders using only calcium silicate powders as an excipient, because the calcium silicate powders are light substance. However, granules having an appropriate loose bulk density and particle size distribution can be obtained by adding a starch, a saccharide, or a mixture of a starch and a saccharide to granulate the powders as described above.

The thus obtained granules by the granulation has a loose bulk density of 0.20 to 0.80 g/ml and preferably 0.30 to 0.60 g/ml.
Further, by compression molding the granules, a tablet with an appropriate diameter and appropriate weight for a demander can be obtained.
As the starch to be used, any known starches can be used without limitation. For Example, there are starches such as a wheat starch, a rice starch, a corn starch, a potato starch, and a tapioca starch, and dextrins obtained by subjecting those starches to an enzymolysis, an acid decomposition, or an alkali decomposition, or by a combination of those decompositions.

Preferable examples are dextrins. As the dextrin, any one of maltodextrin, achrodextrin, erythrodextrin, and amylodextrin can be used. In these dextrins, maltodextrin is preferred.
As the saccharide to be used, any known saccharides can be used without limitation. For example, there are glucose, maltose, and the like. Further, saccharides to be used include sugar alcohols obtained by hydrogenerating those saccharides.

Preferable saccharide is sugar alcohol. As the sugar alcohol, any one of sorbitol, erythritol, xylitol, and maltitol can be used.
The mixing ratio of the starch, the saccharide, or the mixture of the starch and the saccharide is 10 to 30 parts by weight and preferably 15 to 20 parts by weight with respect to 10 parts by weight of porous calcium silicate powders in view of an effect of improving granulating property.

The granulation of a mixture of the functional component-holding calcium silicate powders and a starch or a saccharide is conducted under the presence of a definite amount of water and/or the organic solvent. Both of a spray granulation and an agitation granulation can be employed to granulation process, but an agitation granulation is preferred. The agitation granulation is performed in various manners, and there is agitation granulation by a rotating arm in the top of the container, by a rotor on the bottom of the container, or by agitation in the different direction in addition to the above agitation. In particular, Vertical Granulator (trade name: manufactured by Powlex Co., Ltd.) is preferably used.

The amount of water and/or organic solvent to be used in the granulation is 0.6- to 0.8-fold with respect to the weight of mixed powders of the functional component-holding calcium silicate powders and starches or saccharides.
In the present invention, the thus obtained granules are dried to remove water or the organic solvent included in the granules. The drying temperature may be appropriately selected by taking stability of mixed functional substance into consideration, and is 20 to 90°C, preferably 25 to 75°C, and more preferably 30 to 60°C.

The dried granules are formed into a tablet by regulating the particle size, adding a polymer additive and lubricant thereto, and compression molding the mixture. As a tabletting machine to be used, any one of known tabletting machines can be used without limitation.
As the polymer additive to be used, a polymer additive generally used in producing granules and tablets can be used. For example, there are celluloses such as a crystalline cellulose, a hydroxypropyl cellulose, and a hydroxypropylmethyl cellulose, and synthetic polymers such as polyvinylpyrrolidone, and natural polymers such as an arabic rubber, agar, and pullulan.

Usually, One or more polymer additives are used in combination. According to their additive purpose, each of the polymer additives can be independently used as a excipient, a binder, or a disintegrant.
The thus produced tablet may be appropriately put into a coating step such as a film coating or a sugar coating.
There are described examples below.

### First Example

A granulated product including a *Serenoa repens* extract as a liquid extract derived from a natural product was produced as follows.
18 parts by weight of *Serenoa repens* extract were added to 21.6 parts by weight of ethanol to prepare a dispersion solution.
27 parts by weight of calcium silicate (FLORITE R described above) were sieved and put in a granulator (Vertical Granulator), and subsequently, the *Serenoa repens* extract-dispersed solution and 54 parts by weight of water were put therein, followed by mixing for 5 minutes.

Next, 49 parts by weight of dextrin were added to the mixture, and the whole was granulated by agitation for 5 minutes. After the granulation, regulation of particle diameters and drying were processed. The obtained granules had a loose bulk density of 0.43 g/ml.
Further, 5 parts by weight of agar and 1 part by weight of sucrose fatty acid ester were added to the granules to produce granules for tabletting. A round tablet with a diameter of 8 mm, a weight of 300 mg, and a hardness of 9.4 kgf was obtained with a tabletting machine.

### Second Example

A granulated product including a *Serenoa repens* extract as a liquid extract derived from a natural product was produced as follows.
18 parts by weight of *Serenoa repens* extract were added to 21.6 parts by weight of ethanol to prepare a dispersion solution.
27 parts by weight of calcium silicate (FLORITE R described above) were sieved and put in a granulator (Vertical Granulator), and subsequently, the *Serenoa repens* extract-dispersed solution and 54 parts by weight of water were put therein, followed by mixing for 5 minutes.

Next, 49 parts by weight of sorbitol were added to the mixture, and the whole was granulated by agitation for 5 minutes. After the granulation, regulation of particle diameters and drying were processed. The obtained granules had a loose bulk density of 0.47 g/ml.
Further, 5 parts by weight of agar and 1 part by weight of sucrose fatty acid ester were added to the granules to produce granules for tabletting. A round tablet with a diameter of 8 mm, a weight of 300 mg, and a hardness of 12.8 kgf was obtained with a tabletting machine.

### Third Example

A granulated product including a *Serenoa repens* extract as a liquid extract derived from a natural product was produced as follows.
18 parts by weight of *Serenoa repens* extract were added to 21.6 parts by weight of ethanol to prepare a dispersion solution.
27 parts by weight of calcium silicate (FLORITE R) were sieved and put in a granulator (Vertical Granulator), and subsequently, the *Serenoa repens* extract-dispersed solution and 54 parts by weight of water were put therein, followed by mixing for 5 minutes.

Next, 49 parts by weight of xylitol were added to the mixture, and the whole was granulated by agitation for 5 minutes. After the granulation, regulation of particle diameters and drying were processed. The obtained granules had a loose bulk density of 0.43 g/ml.
Further, 5 parts by weight of agar and 1 part by weight of sucrose fatty acid ester were added to the granules to produce granules for tabletting. A round tablet with a diameter of 8 mm, a weight of 300 mg, and a hardness of 9.7 kgf was obtained with a tabletting machine.

### Fourth Example

A granulated product including a *Serenoa repens* extract as a liquid extract derived from a natural product was produced as follows.
18 parts by weight of *Serenoa repens* extract were added to 21.6 parts by weight of ethanol to prepare a dispersion solution.
27 parts by weight of calcium silicate (FLORITE R) were sieved and put in a granulator (Vertical Granulator), and subsequently, the *Serenoa repens* extract-dispersed solution and 54 parts by weight of water were put therein, followed by mixing for 5 minutes.

Next, 49 parts by weight of maltitol were added to the mixture, and the whole was granulated by agitation for 5 minutes. After the granulation, regulation of particle diameters and drying were processed. The obtained granules had a loose bulk density of 0.46 g/ml.
Further, 5 parts by weight of agar and 1 part by weight of sucrose fatty acid ester were added to the granules to produce granules for tabletting. A round tablet with a diameter of 8 mm, a weight of 300 mg, and a hardness of 25.0 kgf was obtained with a tabletting machine.

### Fifth Example

A granulated product including a *Serenoa repens* extract as a liquid extract derived from a natural product was produced as follows.
18 parts by weight of *Serenoa repens* extract were added to 21.6 parts by weight of ethanol to prepare a dispersion solution.
27 parts by weight of calcium silicate (FLORITE R) were sieved and put in a granulator (Vertical Granulator), and subsequently, the *Serenoa repens* extract-dispersed solution and 94.5 parts by weight of water were put therein, followed by mixing for 5 minutes.

Next, 49 parts by weight of erythritol were added to the mixture, and the whole was granulated by agitation for 5 minutes. After the granulation, regulation of particle diameters and drying were processed. The obtained granules had a loose bulk density of 0.36 g/ml.
Further, 5 parts by weight of agar and 1 part by weight of sucrose fatty acid ester were added to the granules to produce granules for tabletting. A round tablet with a diameter of 8 mm, a weight of 300 mg, and a hardness of 3.9 kgfwas obtained with a tabletting machine.

### Sixth Example

A granulated product including a *Serenoa repens* extract as a liquid extract derived from a natural product was produced as follows.
18 parts by weight of *Serenoa repens* extract were added to 21.6 parts by weight of ethanol to prepare a dispersion solution.
27 parts by weight of calcium silicate (FLORITE R) were sieved and put in a granulator (Vertical Granulator), and subsequently, the *Serenoa repens* extract-dispersed solution and 94.5 parts by weight of water were put therein, followed by mixing for 5 minutes.

Next, 49 parts by weight of mannitol were added to the mixture, and the whole was granulated by agitation for 5 minutes. After the granulation, regulation of particle diameters and drying were processed. The obtained granules had a loose bulk density of 0.30 g/ml.
Further, 5 parts by weight of agar and 1 part by weight of sucrose fatty acid ester were added to the granules to produce granules for tabletting. A round tablet with a diameter of 8 mm, a weight of 300 mg, and a hardness of 3.0 kgfwas obtained with a tabletting machine.

### Seventh Example

A granulated product including a *Serenoa repens* extract as a liquid extract derived from a natural product was produced as follows.
18 parts by weight of *Serenoa repens* extract were added to 21.6 parts by weight of ethanol to prepare a dispersion solution.
27 parts by weight of calcium silicate (FLORITE R) were sieved and put in a granulator (Vertical Granulator), and subsequently, the *Serenoa repens* extract-dispersed solution and 54 parts by weight of water were put therein, followed by mixing for 5 minutes.

Next, 24.5 parts by weight of erythritol and 24.5 parts by weight of xylitol were added to the mixture, and the whole was granulated by agitation for 5 minutes. After the granulation, regulation of particle diameters and drying were processed. The obtained granules had a loose bulk density of 0.45 g/ml.
Further, 5 parts by weight of agar and 1 part by weight of sucrose fatty acid ester were added to the granules to produce granules for tabletting. A round tablet with a diameter of 8 mm, a weight of 300 mg, and a hardness of 7.4 kgf was obtained with a tabletting machine.

### Eighth Example

A granulated product including a *Serenoa repens* extract as a liquid extract derived from a natural product was produced as follows.
18 parts by weight of *Serenoa repens* extract were added to 21.6 parts by weight of ethanol to prepare a dispersion solution.
27 parts by weight of calcium silicate (FLORITE R) were sieved and put in a granulator (Vertical Granulator), and subsequently, the *Serenoa repens* extract-dispersed solution and 54 parts by weight of water were put therein, followed by mixing for 5 minutes.

Next, 32.7 parts by weight of erythritol and 16.3 parts by weight of xylitol were added to the mixture, and the whole was granulated by agitation for 5 minutes. After the granulation, regulation of particle diameters and drying were processed. The obtained granules had a loose bulk density of 0.43 g/ml.
Further, 5 parts by weight of agar and 1 part by weight of sucrose fatty acid ester were added to the granules to produce granules for tabletting. A round tablet with a diameter of 8 mm, a weight of 300 mg, and a hardness of 6.8 kgf was obtained with a tabletting machine.

### Ninth Example

A granulated product including a *Serenoa repens* extract as a liquid extract derived from a natural product was produced as follows.
9 parts by weight of *Serenoa repens* extract were added to 21.6 parts by weight of ethanol to prepare a dispersion solution.
27 parts by weight of calcium silicate (FLORITE R) were sieved and put in a granulator (Vertical Granulator), and subsequently, the *Serenoa repens* extract-dispersed solution and 67.5 parts by weight of water were put therein, followed by mixing for 5 minutes.

Next, 29 parts by weight of erythritol and 29 parts by weight of xylitol were added to the mixture, and the whole was granulated by agitation for 5 minutes. After the granulation, regulation of particle diameters and drying were processed. The obtained granules had a loose bulk density of 0.49 g/ml.
Further, 5 parts by weight of agar and 1 part by weight of sucrose fatty acid ester were added to the granules to produce granules for tabletting. A round tablet with a diameter of 8 mm, a weight of 300 mg, and a hardness of 11.4 kgf was obtained with a tabletting machine.

### Tenth Example

A granulated product including a *Serenoa repens* extract as a liquid extract derived from a natural product was produced as follows.
4.5 parts by weight of *Serenoa repens* extract were added to 21.6 parts by weight of ethanol to prepare a dispersion solution.
27 parts by weight of calcium silicate (FLORITE R) were sieved and put in a granulator (Vertical Granulator), and subsequently, the *Serenoa repens* extract-dispersed solution and 67.5 parts by weight of water were put therein, followed by mixing for 5 minutes.

Next, 31.3 parts by weight of erythritol and 31.2 parts by weight of xylitol were added to the mixture, and the whole was granulated by agitation for 5 minutes. After the granulation, regulation of particle diameters and drying were processed. The obtained granules had a loose bulk density of 0.53 g/ml.
Further, 5 parts by weight of agar and 1 part by weight of sucrose fatty acid ester were added to the granules to produce granules for tabletting. A round tablet with a diameter of 8 mm, a weight of 300 mg, and a hardness of 15.4 kgf was obtained with a tabletting machine.

### Eleventh Example

A granulated product including lutein as an oily substance was produced as follows.
12 parts by weight of lutein (oil containing 20% lutein) were added to 21.6 parts by weight of ethanol to prepare a dispersion solution.
30 parts by weight of calcium silicate (FLORITE R) were sieved and put in a granulator (Vertical Granulator), and subsequently, the lutein-dispersed solution and 60 parts by weight of water were put therein, followed by mixing for 5 minutes.

Next, 26 parts by weight of erythritol and 26 parts by weight of xylitol were added to the mixture, and the whole was granulated by agitation for 5 minutes. After the granulation, regulation of particle diameters and drying were processed. The obtained granules had a loose bulk density of 0.45 g/ml.
Further, 5 parts by weight of agar and 1 part by weight of sucrose fatty acid ester were added to the granules to produce granules for tabletting. A round tablet with a diameter of 8 mm, a weight of 250 mg, and a hardness of 11.4 kgf was obtained with a tabletting machine.

### Twelfth Example

A granulated product including lutein as an oily substance was produced as follows.
12 parts by weight of lutein (oil containing 20% lutein) were added to 21.6 parts by weight of ethanol to prepare a dispersion solution.
30 parts by weight of calcium silicate (FLORITE R) were sieved and put in a granulator (Vertical Granulator), and subsequently, the lutein-dispersed solution and 60 parts by weight of water were put therein, followed by mixing for 5 minutes.

Next, 26 parts by weight of erythritol and 26 parts by weight of dextrin were added to the mixture, and the whole was granulated by agitation for 5 minutes. After the granulation, regulation of particle diameters and drying were processed. The obtained granules had a loose bulk density of 0.51 g/ml.
Further, 5 parts by weight of agar and 1 part by weight of sucrose fatty acid ester were added to the granules to produce granules for tabletting. A round tablet with a diameter of 8 mm, a weight of 300 mg, and a hardness of 14.4 kgf was obtained with a tabletting machine.

## Claims

1. A granule having a loose bulk density of 0.30 to 0.80 g/ml, comprising:
an oily substance in an amount of 5 to 40 parts by weight;
a porous calcium silicate in an amount of 5 to 35 parts by weight; and
a starch in an amount of 10 to 65 parts by weight.

2. Agranule having a loose bulk density of 0.30 to 0.80 g/ml, comprising:
a liquid substance derived from natural product in an amount of 5 to 40 parts by weight;
a porous calcium silicate in an amount of 5 to 35 parts by weight; and
a starch in an amount of 10 to 65 parts by weight.

3. A granule having a loose bulk density of 0.30 to 0.80 g/ml, comprising:
an oily substance in an amount of 5 to 40 parts by weight;
a porous calcium silicate in an amount of 5 to 35 parts by weight; and
a saccharide in an amount of 10 to 65 parts by weight.

4. Agranule having a loose bulk density of 0.30 to 0.80 g/ml, comprising:
a liquid substance derived from natural product in an amount of 5 to 40 parts by weight;
a porous calcium silicate in an amount of 5 to 35 parts by weight; and
a saccharide in an amount of 10 to 65 parts by weight.

5. Agranule having a loose bulk density of 0.30 to 0.80 g/ml, comprising:
a low-melting substance in an amount of 5 to 40 parts by weight;
a porous calcium silicate in an amount of 5 to 35 parts by weight; and
a saccharide in an amount of 10 to 65 parts by weight.

6. Agranule having a loose bulk density of 0.30 to 0.80 g/ml, comprising:
an oily substance in an amount of 5 to 40 parts by weight;
a porous calcium silicate in an amount of 5 to 35 parts by weight;
a saccharide in an amount of 5 to 33 parts by weight; and
a starch in an amount of 5 to 33 parts by weight.

7. Agranule having a loose bulk density of 0.30 to 0.80 g/ml, comprising:
a liquid substance derived from a natural product in an amount of 5 to 40 parts by weight;
a porous calcium silicate in an amount of 5 to 35 parts by weight;
a saccharide in an amount of 5 to 33 parts by weight; and
a starch in an amount of 5 to 33 parts by weight.

8. A granule having a loose bulk density of 0.30 to 0.80 g/ml, comprising:
a low-melting substance in an amount of 5 to 40 parts by weight;
a porous calcium silicate in an amount of 5 to 35 parts by weight;
a saccharide in an amount of 5 to 33 parts by weight; and
a starch in an amount of 5 to 33 parts by weight.

9. A granule according to any one of claims 1 to 8, which have a bulky density of 0.35 to 0.55 g/ml.

10. A granule according to any one of claims 1 to 8, wherein the porous calcium silicate comprises a gyrolite-type calcium silicate having a petaloid crystal structure.

11. A granule according to any one of claims 1 to 8, wherein the starch comprises a dextrin.

12. A granule according to any one of claims 3 to 8, wherein the saccharide comprises a sugar alcohol.

13. A tablet, which is obtained by compression molding the granules according to any one of claims 1 to 8.

14. A method of producing the granules according to claim 1, comprising:
mixing a solution in which the oily substance is dissolved or dispersed in an organic solvent and the porous calcium silicate to allow calcium silicate powders to adsorb and hold the oily substance; and
granulating a mixed powder composition obtained by mixing the oily substance-holding calcium silicate and the starch.

15. A method of producing the granules according to claim 2, comprising:
mixing a solution in which the liquid substance derived from a natural product is dissolved or dispersed in an organic solvent and the porous calcium silicate to allow calcium silicate powders to adsorb and hold an oily substance; and
granulating a mixed powder composition obtained by mixing the oily substance-holding calcium silicate and the starch.

16. A method of producing the granules according to claim 3, comprising:
mixing a solution in which the oily substance is dissolved or dispersed in an organic solvent and the porous calcium silicate to allow calcium silicate powders to adsorb and hold the oily substance; and
granulating a mixed powder composition obtained by mixing the oily substance-holding calcium silicate and the saccharide.

17. A method of producing the granules according to claim 4, comprising:
mixing a solution in which the liquid substance derived from a natural product is dissolved or dispersed in an organic solvent and the porous calcium silicate to allow calcium silicate powders to adsorb and hold an oily substance; and
granulating a mixed powder composition obtained by mixing the oily substance-holding calcium silicate and the saccharide.

18. A method of producing the granules according to claim 5, comprising:
mixing a solution in which the low-melting substance is dissolved or dispersed in an organic solvent and the porous calcium silicate to allow calcium silicate powders to adsorb and hold an oily substance; and
granulating a mixed powder composition obtained by mixing the oily substance-holding calcium silicate and the saccharide.

19. A method of producing the granules according to claim 6, comprising:
mixing a solution in which the oily substance is dissolved or dispersed in an organic solvent and the porous calcium silicate to allow calcium silicate powders to adsorb and hold the oily substance; and
granulating a mixed powder composition obtained by mixing the oily substance-holding calcium silicate, the saccharide, and the starch.

20. A method of producing the granules according to claim 7, comprising:
mixing a solution in which the liquid substance derived from a natural product is dissolved or dispersed in an organic solvent and the porous calcium silicate to allow calcium silicate powders to adsorb and hold an oily substance; and
granulating a mixed powder composition obtained by mixing the oily substance-holding calcium silicate, the saccharide, and the starch.

21. A method of producing the granules according to claim 8, comprising:
mixing a solution in which the low-melting substance is dissolved or dispersed in an organic solvent and the porous calcium silicate to allow calcium silicate powders to adsorb and hold an oily substance; and
granulating a mixed powder composition obtained by mixing the oily substance-holding calcium silicate, the saccharide, and the starch.

22. Amethod of producing a tablet, comprising:
adding a polymer additive and a lubricant to the granules produced by the production method according to any one of claims 14 to 21; and
then compression molding the resultant granules.
